# EUROPEAN PATENT APPLICATION

(11) **EP 1 398 656 A1**
(43) Date of publication of application: **17.03.2004**
(21) Application number: 02405797.8
(22) Date of filing: 12.09.2002
(51) Int. Cl.: G02B 21/06, G03F 7/20, G01N 33/543

(54) **Surface-plasmon-generated light source and its use**

(71) Applicant: Olimatech Ltd., 8803 Rüschlikon (CH)
(72) Inventor: Martin, Olivier J. F. Prof. Dr., 8803 Rüschlikon (CH)
(74) Representative: Barth, Carl Otto, Dipl.-Ing.

(57) **Abstract**

An apparatus, a method and their use for producing localized, small light sources utilizing surface plasmon are disclosed. An illuminating light (15) is directed at an interface of a surface-plasmon-supporting layer (11) in a stratified structure. This illuminating light excites a surface plasmon (16), which can be interpreted as electron density fluctuations, at one surface of the plasmon-supporting layer. The orientation of the electromagnetic field associated with this surface plasmon is such that depolarization fields are created at specific material interfaces in the system, thereby producing localized light sources (18). The lateral extension of these light sources is determined by the geometry of the interfaces producing them. Thus, light sources with a spatial extension not limited by the Rayleigh criterion can be created. These light sources can have an arbitrary shape and are decoupled from the background associated with the illuminating light. Applications of such localized light sources include, among others, optical lithography, optical data storage, biochips and optical microscopy.

## Description

### Technical Field

The present invention relates to optical sources, more precisely to the localization of light on surfaces or in volumes with one or several dimensions not limited by the Rayleigh criterion.

### Background of the Invention

The localization of light on small dimension surfaces or volumes is of interest for many applications. These applications include, but are not limited to:
- lithography, where the localized fields are used to transfer specific patterns into a photosensitive medium;
- optical data storage, where localized sources are utilized to write or read information in a recording medium;
- biochips and biosensors, where the interaction of light with biological or chemical material provides information thereon;
- microscopy, where the localized field is used to image small dimension samples.

To increase the resolution and information density in these different systems, it is desirable to dispose of light sources localized on arbitrarily small dimensions and not limited by the operation wavelength.

In classical optics, light cannot be focused on dimensions smaller than the Rayleigh limit kλ/NA, where λ is the radiation wavelength, NA the numerical aperture, and k a constant which depends on the type of imaging system. This is described by M. Born and E. Wolf in "Principles of Optics", 6^{th} Ed., Pergamon Press, Oxford, 1980. Within the limits set by the Rayleigh criterion, there are two possible approaches for producing a light source with a smaller extension: either reduce the wavelength λ or increase the numerical aperture NA. In optical lithography for example, the current trend consists in reducing the wavelength by using light sources in the ultra-violet (UV), deep UV, X-rays or even electron beams, see "The international technology roadmap for semiconductors", 2001 Ed., International SEMATECH, 2706 Montopolis Drive, Austin, Texas 78741, USA.

The Rayleigh limit can also be tackled from both sides, by simultaneously reducing the wavelength λ and increasing the numerical aperture NA. This is the case in recent developments for optical data storage, where a short wavelength source in the blue is combined with an immersion lens with a large numerical aperture, see S. Imanishi et al. "Near-field optical head for disc mastering process", Jpn. J. Appl. Phys., Vol. 39, pp. 800-805 (2000).

However, it is possible to completely overcome the Rayleigh limit by using near-field optics, see E. Betzig et al. "Breaking the diffraction barrier: optical microscopy of a nanometric scale", Science vol. 251, pp. 1468-1470 (1991). In near-field optics, the electromagnetic field can be confined to features much smaller than the wavelength. Actually, the near-field can be localized on dimensions that are even independent of the wavelength and solely determined by the topography of the system, see O.J.F. Martin et al. "Dielectric versus topographic contrast in near-field microscopy", J. Opt. Soc. Am. A, Vol. 9, pp. 1801-1808 (1996). Current techniques for realizing such near-fields include the utilization of small apertures in an opaque screen, see B. Hecht et al. "Scanning near-field optical microscopy with aperture probes: Fundamentals and applications", J. Chem. Phys., Vol. 112, pp. 7761-7774 (2000), or the scattering of light at a sharp tip, see F. Zenhausern et al. "Scanning interferometric apertureless microscopy: optical imaging at 10 Angstrom resolution", Science, Vol. 269, pp. 1083-1085 (1995).

From a physical point of view, optical near-field effects can be related to the depolarization of the electromagnetic field, which takes place at any interface between materials having different electromagnetic properties, e.g. refractive index, permittivity, and/or permeability. To fulfill the boundary conditions imposed by Maxwell's equations at the interface, specific components of the incident field can be magnified by an amount proportional to the contrast of the material properties. For example, at an interface between two media 1 and 2 with respective permittivity ε₁, and ε₂ (assuming ε₁> ε₂ and an incident field propagating from medium 1 into medium 2), the electric field component normal to the interface in medium 2 has a magnitude larger (by a factor ε₁/ε₂) than the electric field incident on the other side of the interface, in medium 1, see J.D. Jackson "Classical electrodynamics", 3^{rd}. Ed., Wiley, New York, 1999.

In the context of near-field optics, the transmission properties of metal holes realized in a plasmon-supporting layer have also recently attracted a lot of interest. Several experiments have demonstrated a dramatic enhancement of the field transmitted through such holes, see T.W. Ebbeson et al. "Extraordinary optical transmission through sub-wavelength hole arrays", Nature, Vol. 391, pp. 667-669 (1998). Applications of this scheme for photolithography and near-field optical lithography have been proposed, see P.A. Wolff U.S. Pat. No. 5,789,742 (4 Aug. 1998); T.W. Ebbeson et al. US patent 5 973 316; T.W. Ebbeson et al. US patent 6 052 238; and P.R.H. Stark US patent appl. pub. no. 2002/0056816 of 16 May 2002. Note that all these applications necessitate one or several holes, with subwavelength dimension, in the plasmon-supporting layer, which limits the shape and topology of achievable localized light source. The present invention does not need such holes and thus does not suffer these limitations.

The techniques for structuring the surface of hard or soft materials with protruding or indented features with one or several dimensions in the 10-500 nm range are particularly relevant to the present invention. These techniques are well established and form for example the core of two different nanofabrication techniques: soft lithography and nano-imprint lithography, see S.R. Quake and A. Scherer "From micro- to nanofabrication with soft materials", Science, Vol. 290, pp. 1536-1540 (2000).

Soft lithography utilizes a mask made of soft material, such as siloxane polymers, where sub-100 nm features are defined using a moulding process. A structured master, made typically with electron-beam lithography, that has reliefs in a negative image of the desired pattern, is used as a mould, see Y.N. Xia and G.M. Whitesides "Soft lithography" Angew. Chem. Int. Ed. Engl. vol. 33, pp. 550-575 (1998). Soft lithographic masks have been used as phase masks to reproduce sub-100 nm features photolithographically, see J.A. Rogers et al. "Using an elastomeric phase mask for sub-100 nm photolithography in the optical near field", Appl. Phys. Lett., Vol. 70, pp. 2658-2660 (1997). Similar masks have been used as light coupling structures to pattern a photoresist with features having an arbitrary shape with dimensions in the sub-100 nm range, see H. Schmid et al "Light-coupling masks for lensless, sub-wavelength optical lithography", Appl. Phys. Lett., Vol. 72, pp. 2379-2381 (1998). However, it should be noted that these two lithography techniques are limited by the Rayleigh criterion.

For nano-imprint lithography, the mask is no longer soft, but made of a hard material. In that case, the mask also exhibits sub-100 nm features and can be fabricated using standard electron beam lithography techniques, see L.

Guo et al "Nanoscale silicon field effect transistors fabricated using imprint lithography", Appl. Phys. Lett., Vol. 71, pp. 1881-1883 (1997).

In the context of the present invention and its application for lithography, it must be understood that it is not necessary for a photosensitive layer to be illuminated through its entire thickness. Using for example top surface imaging, it is sufficient that the very top layer of the system is exposed, see V. Rao et al. "Top surface imaging process and materials development for 193 nm and extreme ultraviolet lithography", J. Vac. Sci. Techno). B, Vol. 16, pp. 3722-3725 (1998). Therefore, a limited source, even if it does not extend very deep into the photoresist, does not limit the resolution achievable with a photolithographic process.

Finally, some recent advances in biosensors and biochips relevant to the present invention shall be sketched. A biosensor is a device that consists of a biological recognition element, or bioreceptor, e.g. an antibody, an enzyme, a protein, a nucleic acid, whole cells, tissues or entire organism. Tremendous progress has been achieved over the last ten years in the integration of biosensors onto microchips, to create so-called biochips, see T. Vo-Dinh "Nanosensors and biochips: frontiers in biomolecular diagnostics", Sensors and Actuators B, Vol. 74, pp. 2-11 (2001). Working on a nanometric scale, in addition to increasing the resolution, also provides additional benefits related, for example, to short diffusion distances, high sur-face/volume ratios and small heat capacities.

The interaction of light with biological or chemical material is one of the key diagnostic techniques implemented on a biochip. A biochip can be comprised of only the reactive system, or also integrate excitation (illumination, current, etc...) and detection entities. Since a biochip usually consists of arrays of probes used for different biochemical assays, a localized light source allows increasing the spatial resolution as well as the overall throughput of the chip.

### Summary of the Invention

The present invention contemplates a different technique and principle than using apertures for achieving the localization of light on small dimension surfaces or volumes. It instead uses a surface plasmon generated at a given materials interface. The electromagnetic field associated with this surface plasmon possesses components which are specifically enhanced when they encounter an interface between materials with different electromagnetic properties, e.g. with a differing refractive index, permittivity, and/or permeability. Geometrical elements such as protrusions or material inconsistencies or inclusionsare used to judiciously position such materials interfaces at the locations where the light sources must be created. The extension of these localized light sources is determined by the extension of the said protrusions. In this way, it is possible to localize these light sources on dimensions that are not limited by the Rayleigh criterion.

The practical effect of this result is the application of the invention in fields such as optical lithography, optical data storage, biochips and high resolution optical microscopy.

Further and still other objects of the present invention will become more clearly apparent from the following description in conjunction with the accompanying drawings.

### Brief Description of the Drawings

FIG. 1 illustrates the realization of a localized light source from the interaction of a surface plasmon with the interfaces of protruding elements.
FIG. 2 shows the utilization of additional elements to enhance the coupling of the illuminating field with the surface plasmon.
FIG. 3 describes the possibility of using a stratified surface-plasmon-supporting layer.
FIG. 4 illustrates the utilization of contra-propagating illuminating fields to enhance the properties of the localized light source.
FIG. 5 shows the utilization of light confinement elements embedded in the transmission layer.
FIG. 6 illustrates the possibility of addressing individually the different protrusions of a given source using a complex surface-plasmon-supporting circuit.
FIG. 7 shows the utilization of a localized light source for optical lithography.
FIG. 8 shows a typical field intensity profile obtained from a localized light source.
FIG. 9 describes further means of increasing the performance of a localized light source by using more than one surface-plasmon-supporting layer.
FIG. 10 illustrates the implementation of a localized light source for data storage.
FIG. 11 illustrates the combination of a localized light source with a detection mechanism, for data storage.
FIG. 12 shows the utilization of a localized light source in a biochip.
FIG. 13 illustrates the application of a localized light source for near-field microscopy.

### Detailed Description of Embodiments

In the present context, the term "light source" is used to describe a source of electromagnetic radiation in the visible or any other portion of the electromagnetic spectrum.

The novel and innovative scheme makes use of the interaction of light at specific boundaries or interfaces of the system. At the locations where the light source is to be created, there must be a discontinuity of the electromagnetic properties, e.g. a refractive index, permittivity, and/or permeability change, of the corresponding materials. This discontinuity leads to discontinuities of specific components of the electromagnetic field, which produce the said localized light source. In the present invention, the plasmon resonance is used to create field components which will produce strong discontinuities.

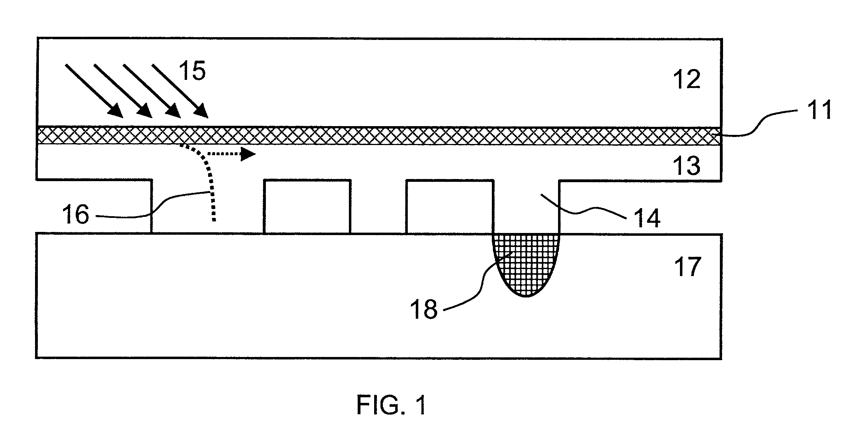

FIG. 1 shows a first embodiment of the present invention. A layer 11 which supports surface plasmons is embedded into a stratified medium composed of a substrate 12 and a transmission layer 13. One or several protrusions 14 are realized on the transmission layer, forming a kind of transmitter-localizer structure together with the transmission layer. The protrusions can be made of the same material as the transmission layer, or of a different material.

The transmission layer and the protrusions, i.e. the transmitter-localizer structure, may have the same or different electromagnetic properties, e.g. refractive index, permittivity, and/or permeability. Further, the transmission layer 13 may not be present, and the protrusions thus placed directly on or in contact with the plasmon-supporting layer 11. Additional material layers, required by the fabrication process or to improve the mechanical properties of the structure may be incorporated in the system; a person skilled in the art should have no problem implementing such alterations or additions.

For visible light operation, the materials which can be used for the plasmon-supporting layer 11 include metals such as gold, silver and copper. Other metals, such as aluminum, can be used in the UV. Metals and metal-oxide mixtures such as indium tin oxide, ITO, can be used in the infrared. Additional materials, including semiconductors, can also be used as surface-plasmon-supporting layer.

The substrate 12 can be made of a dielectric material such as glass or polymer, or of semiconductor material such as silicon or gallium arsenide. The transmission layer 13 or the whole transmitter-localizer structure can be made of a soft material such as a polymer, or a hard material such as glass or a semiconductor. The protrusions 14 can be similarly made of soft or hard materials, either the same or different materials as used for the transmission layer 13.

The protrusions 14 have a height in the order of a fraction of the illumination wavelength. Their lateral dimensions vary according to the shape and size of the light source to be produced. Typically, the protrusions will have at least one lateral dimension smaller than the considered illumination wavelength, e.g. in the order of 20-600 nm for visible and near UV light operation.

At the considered operation wavelength, the electromagnetic properties (e.g. refractive index, permittivity, and/or permeability) of the different materials chosen are such that, when the top surface of the surface-plasmon-supporting layer is illuminated with the external illuminating field 15, a surface plasmon 16 is generated at the bottom surface. This surface plasmon interacts with the protrusions 14 in the structure. Due to the contrast of the electromagnetic properties (e.g. refractive index, permittivity, and/or permeability) between the protrusion 14 and the background 17, depolarization fields 18 are created. These fields form the localized light sources claimed in the present invention. The lateral extension of each light source is determined by that of the corresponding protrusion.

Detailed examples for this first and the subsequently described embodiments follow further below. The usable materials are the same as described above.

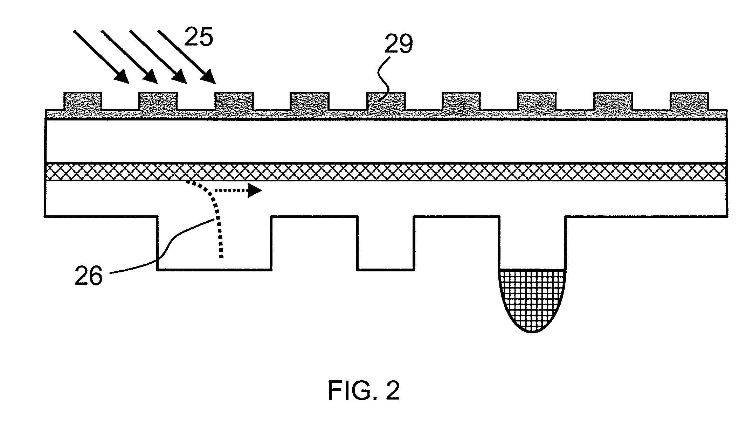

FIG. 2 is a second general embodiment of the present invention, where additional elements, such as a grating 29, or a waveguide, etc., are used to enhance the coupling of the external illuminating field 25 with the surface plasmon 26.

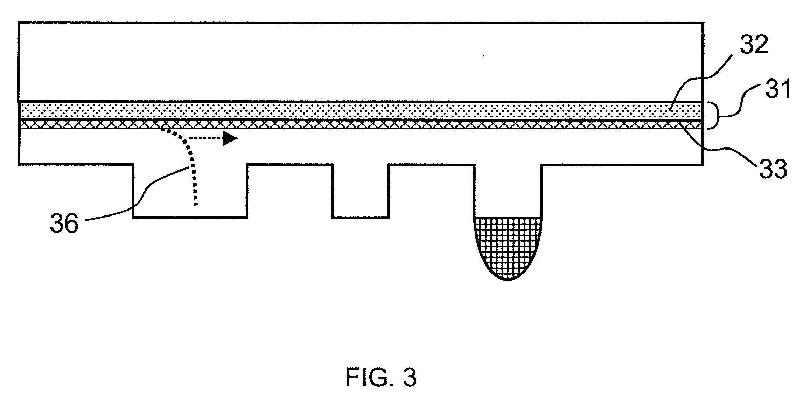

FIG. 3 is a third general embodiment of the present invention, where the plasmon-supporting layer 31 is composed of at least two different materials 32 and 33, the composition and arrangement of which are tuned to obtain a surface plasmon 36 for a specific illumination wavelength.

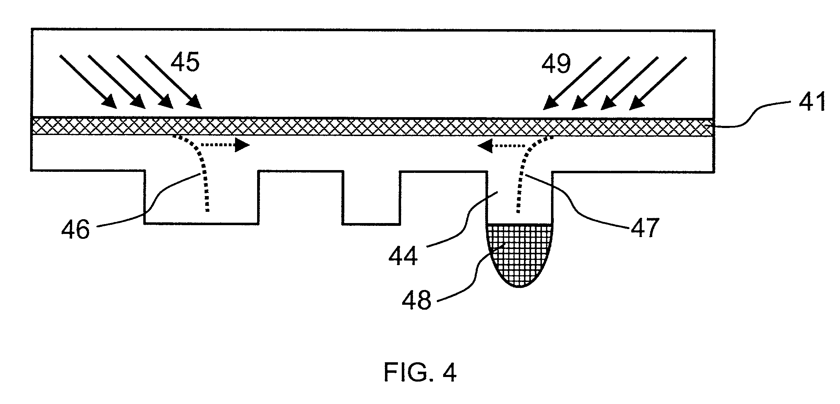

FIG. 4 illustrates the utilization of two contra-propagating external illuminating fields 45 and 49, which generate on the plasmon-supporting layer 41 two contra-propagating surface plasmons 46 and 47, thereby producing a symmetrical light source 48 localized by the protrusions 44. These two illuminating fields may be simultaneously illuminated from different directions, using two or more coherent or incoherent sources. It may also be illuminated in a non-simultaneous way.

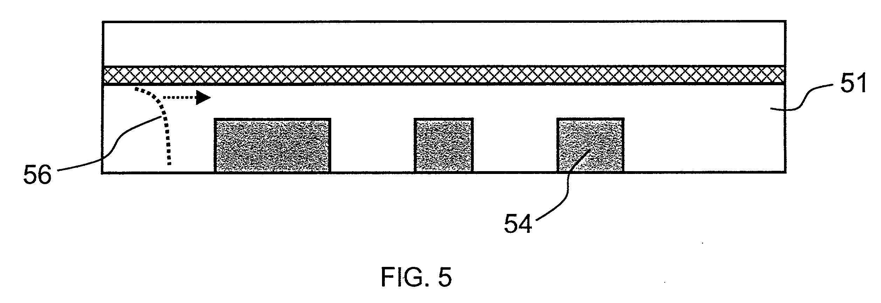

FIG. 5 shows a general embodiment of the invention where the protusions, as e.g. shown in Fig. 1, are replaced by light confining elements 54 embedded in the transmission layer 51, where they interact with the surface plasmon 56. The present invention requires that the electromagnetic properties e.g. refractive index, permittivity, and/or permeability, of the confining elements 54 differ from those of the transmission layer 51. This can be achieved by the inclusion of solid, liquid or gaseous material in the transmission layer 51, by local modification of the transmission layer 51 using e.g. diffusion through its surface, and/or by growing the transmission layer between previously deposited protrusions 54 in order to obtain a flat surface.

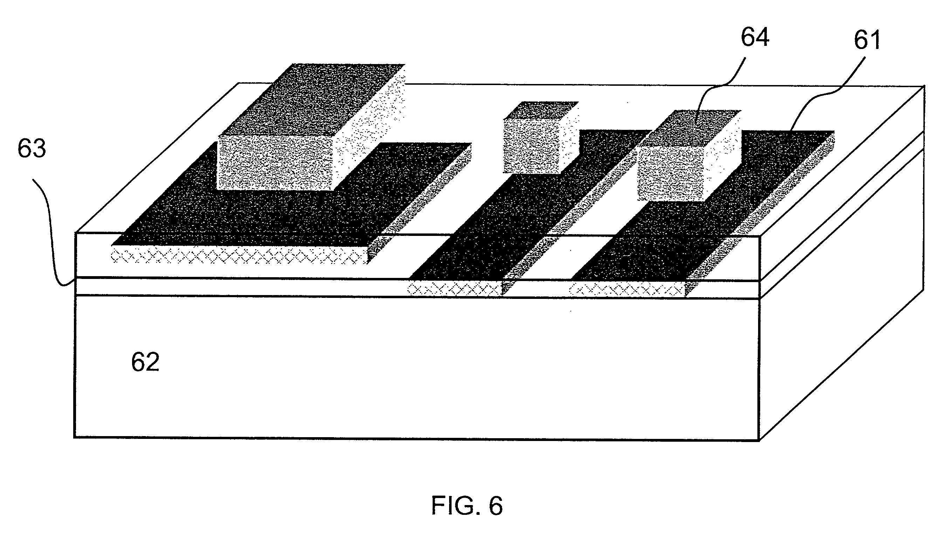

FIG. 6 shows a perspective view of a general embodiment of the invention, where the plasmon-supporting layer, as e.g. shown in Fig. 1, is replaced by plasmon-supporting strips 61, which can be used to individually address one or several protruding elements 64 in the system, using different illuminating fields. Additional material may be placed between adjacent plasmon-supporting strips to obtain a planar structure between the substrate 62 and the transmission layer 63.

The general physical structures described above may be used to advantage in a number of different applications, as described next.

### Optical lithography

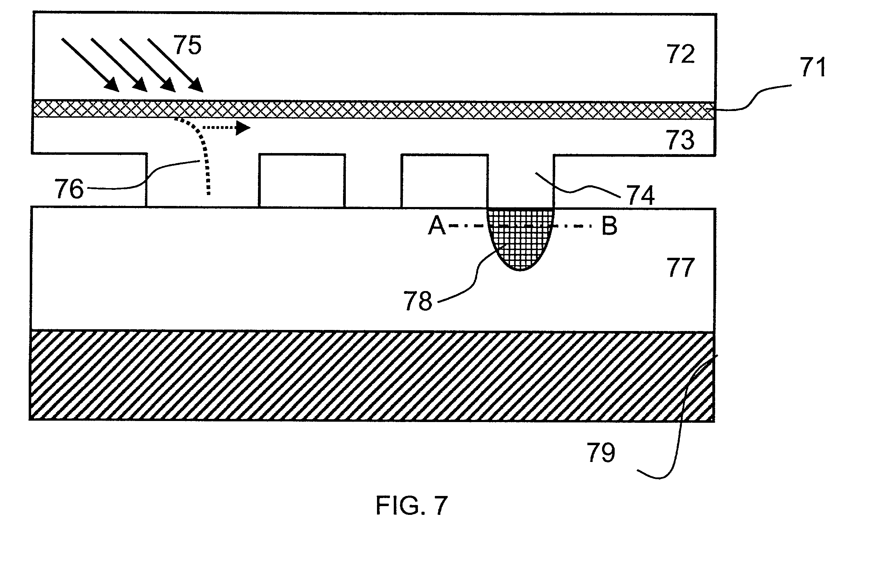

FIG. 7 shows an embodiment of the present invention, with a surface plasmon supporting layer 71, a substrate 72, a transmission layer 73 and protrusions 74, which may be of varying dimensions. This structure shall be called "the mask" in the present context.

The mask is positioned on top of a photosensitive layer 77, which in turn is deposited on an external substrate 79. In the exposition area, contact between the photosensitive layer and the mask occurs only at the protrusion interfaces. For the localization of light 78 at these interfaces between the protruding elements and the photosensitive layer, it is mandatory that their electromagnetic properties, e.g. refractive index, permittivity, and/or permeability, differ. It is further important that the external illuminating light 75 is suited, i.e. has an appropriate wavelength and power, for the creation of the surface plasmon 76 and for the patterning of the photosensitive layer 77.

The lateral shape and size of the protrusions 74 define the lateral shape and size of the exposed portions 78 of the photosensitive layer, and thus the subsequently formed structures. The strength of the light source in the photosensitive layer is determined by the contrast of electromagnetic properties, e.g. refractive index, permittivity, and/or permeability, between the protrusions 74 and the photosensitive layer 77. It is therefore possible to simultaneously expose features of varying dimensions. The contrast between exposed and non-exposed regions depends on the height of the protrusions 74 on the mask. Note that a negative resist can also be used, together with the embodiment shown in FIG 4, which produces an inverted localized source.

The following materials and dimensions may be used for the various parts of the device described above.

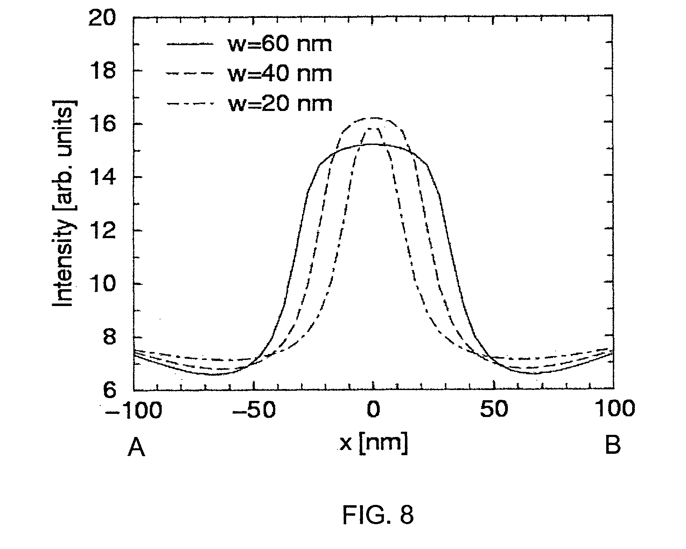

FIG. 8 shows the electric field intensity in the photosensitive layer, along the line AB in FIG 7, for three different protrusion widths: w=20 nm, w=40 nm, and w=60 nm. These distributions were calculated for the illumination wavelength λ=600 nm. The intensity profiles very well reproduce the protrusion widths, although their dimensions are much smaller than the illumination wavelength.

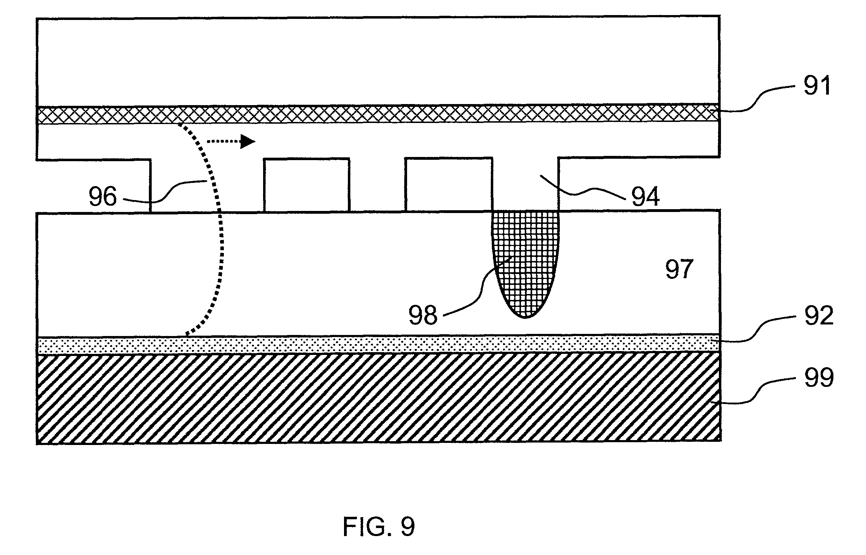

FIG. 9 shows an embodiment of the invention similar to that in FIG. 7, where an additional, second, plasmon-supporting layer 92 is included in the system. For specific distances between the first plasmon-supporting layer 91 and the second one 92, coupled surface plasmon modes 96 can be created in the system. This coupling mechanism can increase the magnitude and the extension of the localized light sources 98 at the interfaces between protrusions 94 and photosensitive layer 97.

Regarding materials and dimensions, the above said applies.

### Optical data storage

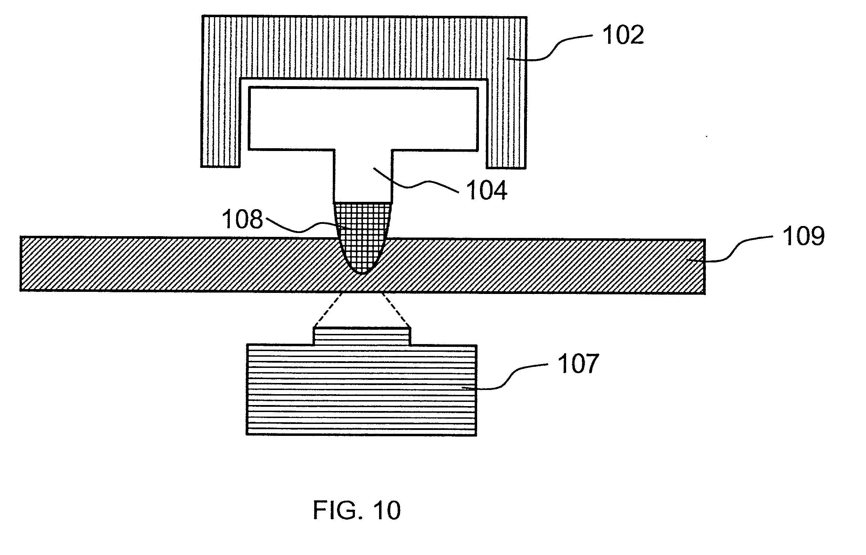

FIG. 10 shows an embodiment of the present invention for data storage. The localized light source 108, which may include one or several protruding elements 104, is incorporated in a storage head 102, which may be fixed or mobile with respect to the recording medium 109. The storage head can incorporate additional mechanisms to track the recording medium and control the distance between the recording medium and the localized light source. This is known in the art and shall not be described here. The localized light source is used to write and/or read information in the recording medium. In this embodiment, the detection mechanism 107 is located on the other side of the recording medium and the writing/reading processes occur in transmission.

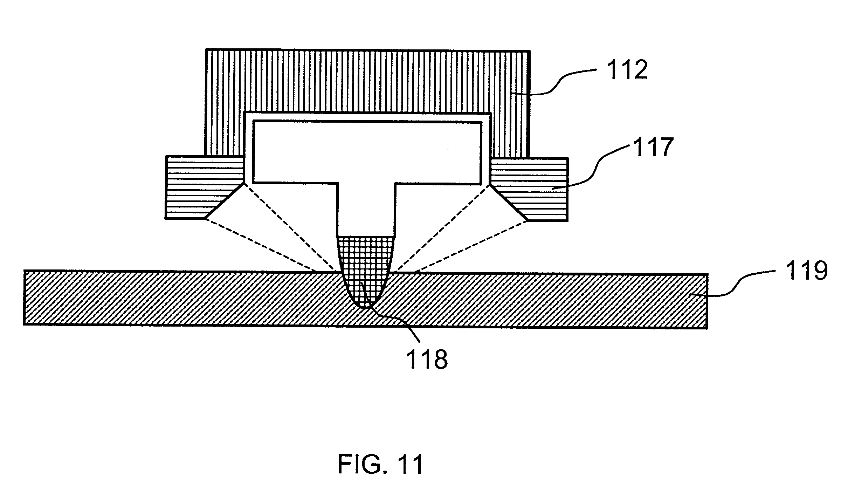

FIG. 11 shows an embodiment of the present invention similar to that in FIG. 10, where the localized light source 118 and the detection mechanism 117 are located on the same side of the recording medium 119. In this case, the detection mechanism 117 may be combined together with the localized illumination source 118, in the recording head 112.

Regarding materials and dimensions, the above said applies.

### Biochips

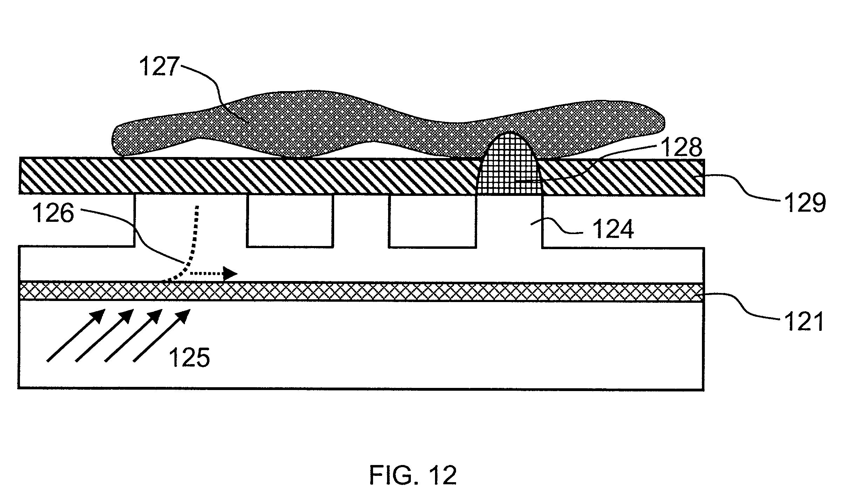

FIG. 12 shows an embodiment of the present invention in a biochip. The surface-plasmon-supporting layer 121 is now at the bottom and the system illuminated from underneath with the external illuminating field 125. The protruding areas 124 and the surface plasmon 126 define localized light sources 128 on the substrate 129, where the biological or chemical material 127 is deposited or embedded. The signal resulting from the interaction of the localized light source with the biological or chemical material may be detected in the far-field or in the near-field of the sample.

Regarding materials and dimensions, the above said applies.

### High resolution optical microscopy

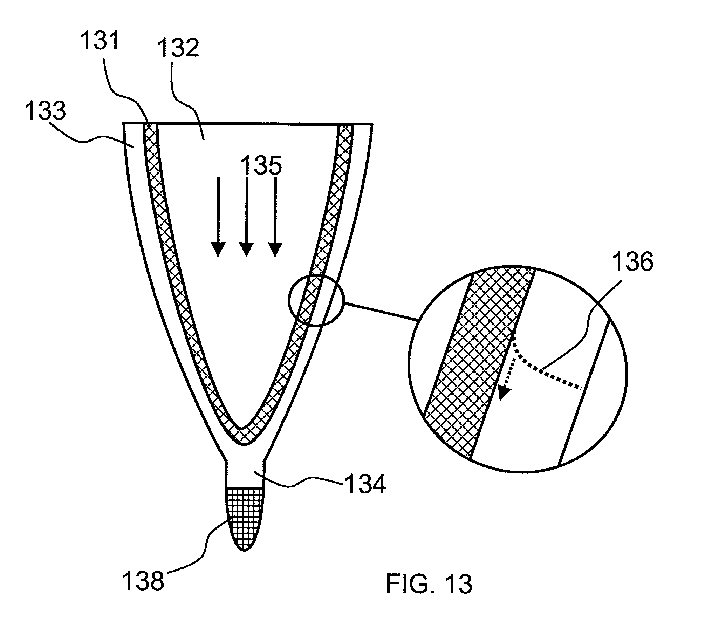

FIG. 13 shows an embodiment of the present invention as a probe for scanning near-field optical microscopy. The substrate 132 may be formed by the core of an optical fiber or a microfabricated structure, on which the surface-plasmon-supporting layer 131 is deposited. When the internal interface of the surface-plasmon-supporting layer is illuminated with the external illuminating field 135, a surface plasmon 136 is generated on the other interface. The interaction of this surface plasmon with one or several protrusions 134 on the transmission layer 133, generate a localized light source 138. The protrusions will have at least one dimension smaller than the illumination wavelength. Regarding the materials for the plasmon-supporting layer, the above said applies.

It is to be understood that the specific embodiments and applications of the invention that have been described are merely illustrative applications of the principles of the invention. The person skilled in the art may make numerous modifications to the described methods and apparatus without departing from the true spirit and scope of the invention.

## Claims

1. A structure for producing a localized light source in a medium, *comprising*
• a source generating incident light (15, 75),
• a surface-plasmon-supporting layer (11, 71 ),
• a transmitter-localizer (13, 14; 73, 74) between said surface-plasmon-supporting layer and said medium (17, 77, 97) providing an interface with predetermined electromagnetic properties,
• wherein said incident light excites a surface plasmon (16, 76) in said surface-plasmon-supporting layer, which plasmon in turn produces said localized light source (48, 78) at said interface.

2. The structure of claim 1, *wherein* the transmitter-localizer between the surface-plasmon-supporting layer (11, 71) and the medium (77, 97) includes means for localizing the electromagnetic field associated with a plasmon generated by said surface-plasmon-supporting layer.

3. The structure of claim 2, *wherein* the means for localizing the electromagnetic field associated with a plasmon consists of or includes one or more protrusions (14, 74) contacting the medium.

4. The structure of claim 2, *wherein* the means for localizing the electromagnetic field associated with a plasmon consists of or includes of one or more inclusions (54) to localize the light source.

5. The structure of any of the preceding claims, *further including* means, in particular a grating (29), for enhancing the generation of surface plasmons by the surface-plasmon-supporting layer (11, 71).

6. The structure of any of the preceding claims, *further including* a substrate (12, 72) carrying the surface-plasmon-supporting layer (11, 71) and the transmitter-localizer (13, 14; 73, 74), and providing a transfer of the incident light (15, 75).

7. The structure of any of the preceding claims, wherein the surface-plasmon-supporting layer (31) is made of two or more different materials (32, 33).

8. The structure of any of the preceding claims, *wherein* a plurality of sources for generating incident light (45, 49) is provided for simultaneous or sequential use.

9. The structure of of any of the preceding claims, *wherein* the surface-plasmon-supporting layer consists or comprises a plurality of patches or strips (61) which are individually addressable.

10. The structure of any of the preceding claims, *further including* one or more additional surface-plasmon-supporting layers for enhancing the localized light source.

11. The structure of any of the preceding claims, *wherein* the various layers and elements of said structure are curved to enable contacting the medium at one particular location.

12. The structure of claim 3 or 4, *wherein* the width and/or length of the means for localizing the generated plasmon, in particular of the protrusion (14, 74), is a fraction of the wavelength of the localized light source, preferably less than about one tenth of said wavelength.

13. The structure of claim 1, *wherein* for visible light operation, the surface plasmon-supporting layer consists of or includes any of gold, silver and/or copper.

14. The structure of claim 1, *wherein* for operation in the UV region, the surface plasmon-supporting layer consists of or includes a metal, preferably aluminum.

15. The structure of claim 1, *wherein* for operation in the infrared region, the surface plasmon-supporting layer consists of or includes a metal and/or a metal-oxyde mixture, preferably indium tin oxide.

16. A method for producing a localized light source in a medium, comprising *the following steps:*
• generating incident light (15, 75),
• exciting a surface plasmon (16) from said incident light in a surface-plasmon-supporting element (11, 71 ),
• transmitting said surface plasmon to a localized interface with predetermined electromagnetic properties between said surface-plasmon-supporting element and said medium, in which interface said surface plasmon produces said localized light source.

17. Use of a structure according to any of the claims 1 to 15 and/or the method according to claim 16 for optical lithography.

18. Use of a structure according to any of the claims 1 to 15 and/or the method according to claim 16 for optical data storage.

19. Use of a structure according to any of the claims 1 to 15 and/or the method according to claim 16 in or for biochips.

20. Use of a structure according to any of the claims 1 to 15 and/or the method according to claim 16 for high resolution optical microscopy.
